Europäisches Patentamt

European Patent Office

Office européen des brevets

(11) Publication number: **0 141 648**
**B1**

(12) EUROPEAN PATENT SPECIFICATION

(45) Date of publication of patent specification: **25.01.89**

(21) Application number: **84307507.8**

(22) Date of filing: **31.10.84**

(51) Int. Cl.⁴: **G 01 N 33/52,** G 01 N 33/72,
G 01 N 33/68, C 12 Q 1/54,
C 12 Q 1/28

(54) Reflective particle-containing analytical device.

(30) Priority: **07.11.83 US 549350**

(43) Date of publication of application:
**15.05.85 Bulletin 85/20**

(45) Publication of the grant of the patent:
**25.01.89 Bulletin 89/04**

(84) Designated Contracting States:
**BE CH DE FR GB IT LI NL SE**

(56) References cited:
EP-A-0 016 387
EP-A-0 072 957
EP-A-0 103 288
EP-A-0 114 403
WO-A-79/01081
US-A-4 381 921
US-A-4 430 436

(73) Proprietor: TECHNICON INSTRUMENTS
CORPORATION
511 Benedict Avenue
Tarrytown, New York 10591 (US)

(72) Inventor: Kumar, Anand
1 Duelk Avenue
Monroe New York 10950 (US)
Inventor: Leong, Koon-Wah
71 Van Cortlandt Avenue
Ossining New York 10562 (US)

(74) Representative: Wain, Christopher Paul et al
A.A. THORNTON & CO. Northumberland House
303-306 High Holborn
London WC1V 7LE (GB)

Courier Press, Leamington Spa, England.

## Description

This invention relates generally to the field of fluid analysis by analytical devices or elements and, more particularly, to devices which are contacted with fluids in which the constituent(s) are to be determined and a detectable change is effected by chemical or other reaction.

The first type of such device which was developed had an irregular, fibrous reagent layer, such as paper. The thickness of the layer was not critical, but needed only to be sufficient to render the reaction layer opaque, e.g. it could be of infinite optical thickness. Analyte concentration was semiquantitatively determined by a visual comparison of the diffuse reflectance signal with a color block chart (see, for example, U.S. Patents Nos. 3,012,976; 3,164,934; and 3,485,587; and Walter, *Anal. Chem. 55*: 498A (1983)). The irregularity of these types of elements severely limited the quantitation which could be achieved. Even the introduction of photometric instrumentation has not overcome this fundamental limitation.

The art has evolved such that most "dry chemistry" elements are now monitored by reflectance methods and include a plurality of layers in various laminar configurations. Each of these is a discrete, separate layer which performs one or more, but less than all, of the necessary functions and is bound to the other layers. The one or more reagent layers incorporates a composition which usually includes all the reagents necessary for analysis of a particular body fluid constituent, therapeutic drug or other analyte in order to provide a measurable response. See Walter, et al., *supra* and also Shirey, Clin. Biochem., *16*: 147—155 (1983). In such elements, incident light passes through the translucent reagent layer(s) where it is absorbed to an extent determined by the analyte/reagent composition. This requires that the reagent layer(s) be uniform and of precisely controlled thickness. A smooth, opaque reflective layer is laminated to the opposite side of the reagent layer. This reflects the unabsorbed light back through the reagent layer to a detector.

One of the simplest analytes for determination in conventional chemistry systems has been albumin. In "dry chemistry" even this analyte requires a multilayer element. Wu, U.S. Patent No. 4,230,456 discloses a multilayer analytical element for albumin which includes a spreading layer, a subbing layer and a reagent layer. The reagent layer contains a complex of anilinonaphthalene sulfonic acid (ANS) and polyvinylalcohol (PVA). Sample albumin passes into the spreading layer but not normally into the reagent layer. Nonetheless, it is said that this albumin causes release of the ANS from the complex into the spreading layer. It is also said that, in use, when albumin is quantified by measuring formation of a complex of albumin and detectable species, it is desirable to use a multilayer analytical element having a transparent spreading layer and opaque reagent layer and that any suitable opacifying agent, such as titanium dioxide, can be used. The method described for reading the result is to pass the element through a zone in which suitable spectrophotometry apparatus directs a beam of energy, such as light, through the support and the reagent layer. It is unclear how this would be operative where the detectable species migrates to a separate layer on the opposite side of the opaque reagent layer.

In yet another type of device, reaction layers have been formed of hollow microspheres or beads and the necessary reagents incorporated within their interior. This approach is disclosed *inter alia* in U.S. Patents Nos. 3,092,463; 3,926,732; 3,993,451; 4,129,417; 4,356,149; and WO 79/01081. For example, Siddigi, WO 79/01081, discloses a test device made of an inert support covered with a layer of polymer beads which contain reagent and in which the color reaction takes place. By way of further example, Kitajima, et al, U.S. Patent No. 4,356,149, disclose a multilayer element in which the reaction layer is of an oil-in-water type dispersion wherein the reagent is contained in hydrophobic particles dispersed in a hydrophilic binder. The multilayer element disclosed also includes a radiation-blocking or light reflection layer between the reagent layer and an aqueous liquid sample-spreading layer.

Pierce, et al, U.S. Patents Nos. 4,258,001 and 4,381,921 (a division of 4,258,001) reflect a further development in the field of multilayer analytical elements which emphasizes a complex three-dimensional lattice structure. Overall, it applies this structure in the type of multilayer elements described above. It also alludes to the use of this structure apart from spreading, reflective or other layers. The only two analytical examples of this lattice structure, apart from other layers, are limited to "qualitative" results for glucose determination over a narrow concentration range and application to hemoglobin analysis for which no data in differentiating hemoglobin concentrations is reported. The three-dimensional lattice structure is formed of heat stable, non-swellable organopolymeric particles and adhesive for these particles. A large portion of the particle surface area is free from adhesive and can have reagents thereon. Preferably, these reagents are chemically bonded to the polymer units of the particles. Although particle size and shape can vary, they are preferably uniform and spherical. The particles typically comprise at least about 25 weight percent, and preferably 50 weight percent or more, of the organo-polymeric material. In many embodiments, these particles are composed entirely, i.e. 100 weight percent, of such organo-polymeric material. The remainder of these particles can be composed of other addenda, for example, colorants such as pigments or dyes; radiation-blocking agents including colorants and other opacifying addenda; fluors; fillers; magnetic addenda, e.g. magnetite; and the like, provided the requisite impermeability and non-swellability properties of the particles are maintained, and the addenda do not interfere with the analysis to be carried out. Thus, the reference teaches that high percentages of organo-polymeric particles are necessary because only they form the three-dimensional lattice.

Thus, the earliest reagent-impregnated paper strips have been limited in precision and use in quantitation whereas the quantitation achieved by multilayered elements has been at the cost of increased complexity and susceptibility to fine variation in manufacturing specifications. The encapsulation of reagents has neither reduced this complexity nor reduced the susceptibility of readings to interference. The preparation of elements formed of complex three-dimensional, organo-polymeric lattices does not reduce the complexity of manufacture nor does it impart satisfactory reflectance characteristics to the described layer.

In contrast to the reagent matrices or elements previously described, the present invention provides the combination of a complete reagent system and a reflective component in a single layer which can be read by diffuse reflectance. The adverse influence of interfering particulate material and sample turbidity is avoided. In addition, the restrictive requirements of uniformity and precise thickness of the matrix are avoided in that light is not required to pass completely through the reagent in order to be reflected for detection. Simplicity in manufacture is combined with multi-functional capacity.

EP—A—0016387 describes a diagnostic device for detecting analytes in liquids which device comprises a water-resistant film composed of a film-former and containing a film opener in the form of fine insoluble inorganic or organic particles. The film also includes one or more reagents for reaction with the analyte of interest to provide a detectable response thereto.

A device according to the present invention consists essentially of a single, integral matrix comprising: (a) a plurality of solid, reflective, inorganic particles (22); (b) each of said particles being coated with at least one substance (24) to provide a detectable response to said analyte; and (c) a binder (26) which is selectively permeable to said analyte or a reaction product thereof; characterised in that the device comprises two analyte responsive components, the second of which is said at least one substance coated on said particles and the first of which is separated therefrom by said binder, and wherein said first component is responsive to the analyte to form a reaction product, and the second component effects a detectable response to the reaction product.

Preferably, the particles are non-spherical and have a refractive index greater than about 1.6.

A device of the invention is completely effective in the absence of other elements or structures to provide a quantitative detectable response to the presence of the analyte under assay.

The invention also provides a method of determining an analyte by contacting the device with a sample suspected of containing the analyte and observing any detectable response. Various embodiments, which include or use the device and/or method are also contemplated. For example, the test material can be provided as part of a test kit. The kit comprises the packaged combination of one or more devices or containers of them in any of a variety of physical forms.

In order that the invention may be more fully understood, reference is made to the accompanying drawings, in which:

Figure 1 is a schematic side view of one embodiment of device of the invention.

Figure 2 is a graphical illustration of the data resulting from the experiments described in Example I.

Specific terms in the following description which refer only to a particular embodiment are exemplary of all of the embodiments unless otherwise indicated.

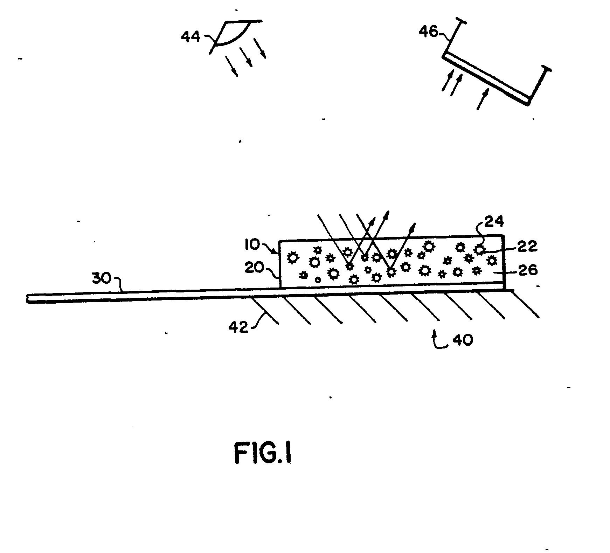

Fig. 1 illustrates a device of the present invention as it can be used with a reflectance instrument. The device 10 has an analysis layer 20. Analysis layer 20 has reflective particles 22 having reagent 24 associated with their surface in the device as illustrated. They are maintained in position by binder 26. Device 10 also has a carrier 30 which takes no part in the analysis, and is provided only for ease of handling. Liquid sample is applied to device 10 to react with reagent 24 in analysis layer 20. Device 10 is positioned in an instrument 40 on base 42. The instrument has a light source 44 which directs light on and into analysis layer 20. A detector 46 detects the amount of reflected light and the instrument reports this as a function of analyte concentration.

Sample fluids on which tests are performed include biological, physiological, industrial, environmental, and other types of liquids. Of particular interest are biological fluids such as serum, plasma, urine, cerebrospinal fluid, saliva, milk, broth and other culture media and supernatants as well as fractions of any of them. Physiological fluids of interest include infusion solutions, buffers, preservative or antimicrobial solutions and the like. Industrial liquids include fermentation media and other processing liquids used, for example, in the manufacture of pharmaceuticals, dairy products and malt beverages. Other sources of sample fluid which are tested by conventional methods are contemplated as within the meaning of this term as used and can, likewise, be assayed in accordance with the invention.

The solid, reflective, inorganic particles used are optically opaque granular or particulate matter which have no reagent or substantial internal void volume and are chemically inert to the sample, reagents and reaction for which the device is intended. Within this limitation, it is desirable to select particles which are not light absorbing at the detection wavelength and have as high a refractive index as possible, preferably above about 1.6. Preferably, the particles are of irregular shape and have a diameter of from 0.1

µm to 200 µm. The combination of high refractive index and irregular particle shape enhances the scattering of incident radiation, thereby increasing analytical signal in a diffuse, reflectance colorimetric mode. Particles which are most preferred include basic lead hydroxide, basic lead carbonate, oxides such as titanium dioxide, zinc oxide and magnesium oxide, and sulfates such as barium sulfate, calcium sulfate and strontium sulfate. Other materials which can be used include silica, calcite, silicates and aluminosilicates, zinc sulfide, diatomaceous earth, clay or mixtures thereof.

The composition can be such that the analyte-responsive substance comprises a first component which is responsive to the analyte to form a reaction product and a second component which is separated from the first component by the binder and effects a detectable response to the reaction product. Preferably the second component is associated with the surface of the particles. In one preferred embodiment the first component is reactive with the analyte to produce hydrogen peroxide and the second component includes a peroxidatively active substance, such as peroxidase, and a redox indicator. For example, where the analyte is cholesterol, the first component includes cholesterol oxidase and cholesterol ester hydrolase. Where the analyte is uric acid, the first component includes uricase.

The term "peroxidatively active substance" defines the precise chemical activity of the substances contemplated. A peroxidase is an enzyme which will catalyze a reaction wherein hydrogen peroxide oxidizes another substance. The peroxidases are generally proteins which incorporate iron porphyrin moieties. Peroxidase occurs in horseradish, potatoes, figtree sap and turnips (plant peroxidase); in milk (lacto peroxidase); and in white blood corpuscles (verdo peroxidase); also it occurs in microorganisms and may be produced by fermentation. Certain synthetic peroxidases, such as those disclosed by Theorell and Maehly in *Acta Chem. Scand., 4*: 422—434 (1950), are also satisfactory for use in $H_2O_2$ detection systems. Less satisfactory are such substances as hemin, methemoglobin, oxyhemoglobin, hemoglobin, hemochromogen, alkaline hematin, hemin derivatives, and certain other compounds which demonstrate peroxidative or peroxidase-like activity, namely, the ability to catalyze the oxidation of another substance by means of hydrogen peroxide and other peroxides. Other substances which are not enzymes but which demonstrate peroxidative activity are: iron sulfocyanate, iron tannate, ferrous ferrocyanide, chromic salts (such as potassium chromic sulfate) absorbed on silica gel, etc.

The redox indicators contemplated include the many which are known to those skilled in the art. These include benzidine, o-toluidine, 3,3',5,5' - tetramethylbenzidine, 2,7 - diaminofluorene, 3 - methyl - 2 - benzothiazolinone hydrazone, optionally with its known couplers, 4 - aminoantipyrine, optionally with its known couplers and other phenazones and pyrones.

Binders which are suitable for use must be permeable to the analyte or a reaction product thereof. The particles and analyte-responsive substance or reagent are dispersed, usually uniformly, in the binder material. The binder comprises a macromolecular substance such as proteins, gums, waxes or, preferably, polymers. The polymer can be, for example, cellulose acetate, polyvinyl chloride, nylon, starch, polycarbonate, polyvinyl alcohol, polyvinyl acetate, polyvinyl pyrrolidone, polyurethane, cellulose, cellulose nitrate or cellulose acetate butyrate. For example, the binder content of each 100 ml of the composition which is cast to form the analytical layer can include 3—9 grams (g) cellulose acetate for each 100 ml of the composition to be formed into a layer.

The test device or analytical element consists essentially of a single integral matrix formed of a composition as described above. The single zone of this composition is all that is required for performance of a complete analysis. This provides freedom from the requirement of multiple distinct spreading reagent, reflection and other layers. The integral analytical elements can be provided on a support for ease of handling. Preferred supports include those of polystyrene or similar plastics. The support can be opaque, translucent or transparent to light or other energy. The devices of the present invention can be made by suitable techniques such as printing or spraying the composition onto a carrier or incorporating the solutions into film-forming liquids and allowing the combination so prepared to set or solidify, thereby imparting dimensional stability. A suitable casting solvent can include, for example, methylene chloride, ethanol and isopropanol. Such coating can be accomplished by hand, using a blade coating device, or by machine, using techniques such as dip or bead coating. The thickness of the element and its degree of permeability are widely variable and depend on actual usage. Dry thicknesses of from about 5 microns to about 200 microns have been convenient, although a broader range of element thickness may be preferable in certain circumstances.

It can be advantageous to incorporate one or more emulsifier or surfactant materials, such as anionic and non-ionic surfactant materials, in the analytical element. They can, for example, enhance coatability of composition formulations and enhance the extent and range of wetting in elements that are not easily wetted by liquid samples in the absence of an aid such as a surfactant. For example, each 100 ml of the composition which is formed into elements can include 0.4—9 grams of surface-active agents like the fatty acid partial esters of sorbitol anhydrides. It can also be desirable to include materials that can render nonactive in the analysis of choice, by chemical reaction or otherwise, materials potentially deleterious to such analysis.

As previously noted, many of the reagent systems which can be used provide, or can be readily adapted to provide, a detectable response, such as a color change, related to the presence or amount of the analyte under assay in the liquid sample. The element of the invention provides enhanced electro-magnetic radiation signals such as a change in light reflectance in the visible spectrum, thereby producing a visible color change, or a change in light reflectance outside the visible range such as in the ultraviolet range or infrared range. The detectable response is one which can be observed through the senses directly or by use of ancillary detection means, such as a spectrophotometer or other sensing means. After the analytical result is obtained as a detectable change, it is measured, usually by passing the test element through a field in which suitable apparatus for reflectance measurement is provided. Such apparatus serves to direct a beam of energy, such as light, which is then reflected from the element back to a detector. Generally, electro-magnetic radiation in the range of from about 200 to about 900 nm has been found useful for such measurements.

The following working Example illustrates the present invention. Standard commercially available reagent grade chemicals were used whenever possible. "Span", "Ansilex", "Triton", "Pentex", "Solitron" and "InfraAlyzer" are trade marks.

Example I

Serum cholesterol concentration is one of the most frequently performed tests in clinical chemistry. Normal values for serum cholesterol are considered to be from about 144 to about 315 mg/dl of serum. Higher levels are associated with plaque formation on arterial walls. Enzymatic methods employing cholesterol oxidase, cholesterol esterase, peroxidase and a chromogenic indicator are generally used for the determination of cholesterol in clinical assays. This example reports experiments in which such an enzymatic method is applied in the preparation and use of the analytical element of the invention.

A 1.0 gram (g) portion of cellulose acetate (39.8% acetylation) was added to a mixture containing 0.5 g Span 20 emulsifier (ICI United States, Inc., Wilmington, DE), 14 milliliters (ml) methylene chloride, 4 ml absolute ethanol, and 3 ml isopropanol. The cellulose acetate was allowed to dissolve therein for at least 2 hours. Then, 19 g titanium dioxide and 1 g Ansilex clay (Englehard Minerals & Chemical Corporation) were added and shaken vigorously. To this mixture a solution containing 3000 IU horseradish peroxidase, 0.1 g bovine serum albumin, 0.2 g 4-aminoantipyrine, 0.3 g sodium p-hydroxybenzoate, 10 milligram (mg) disodium EDTA, 4 mg potassium azide and 2 ml 0.2 molar (M) phosphate buffer (pH 6.8) was added and the mixture was agitated thoroughly for 15 seconds.

A 7 ml volume of the above-prepared material was placed on the uncoated side of a gel bond transparent polyester film (Marine Colloids, Rockland, ME) and spread to a depth of 0.5 millimeter (mm) using a conventional doctor blade. A reagent zone of approximately 180 square centimeters of 150 µm thickness was produced. The film was air dried for approximately 30 minutes.

Onto this film a solution containing 1136 IU cholesterol esterase, 744 IU cholesterol oxidase, 5 mg sodium cholate, 1 mg EDTA, 1 mg bovine serum albumin, 15.6 mg sodium azide, 2.4 mg Triton X-100, and 2.4 ml 0.2 molar (M) phosphate buffer (pH 6.8) was impregnated by surface coating. The reagent impregnated film was air dried for approximately 30 minutes and stored in a desiccator for 2 hours. The dry coated polyester film was then cut into 1.27 centimeter diameter circular discs. Twelve solutions containing cholesterol concentrations, ranging from 40 mg/dl to 650 mg/dl, were prepared from Pentex cholesterol concentrate (Miles Laboratories, Elkhart, IN).

Analysis of each test solution was performed by pipetting 100 microlitres of the particular test solution under assay on a separate one of the reagent-containing film discs prepared above. Each disc was thereafter spun at 7000 rpm on a Solitron spinner for 10 seconds. Each of the discs was maintained at room temperature for about 5 minutes and thereafter, the amount of cholesterol in each solution was quantified at 500 nanometers (nm) on an InfraAlyzer reflectance colorimeter (Technicon Instruments Corporation, Tarrytown, NY).

This procedure provided data in the form of reflectance units (R) which were mathematically converted by the Kubelka-Monk equation, $(1-R)^2/2R = K/S$, wherein K is the absorption coefficient and S is the scattering coefficient of the particular reflecting medium. Kartum, *Reflectance Spectroscopy*, pgs. 106—111, Springer-Verlag, NY (1969). K/S is a function of the colored species concentrations and increases with the analyte concentration.

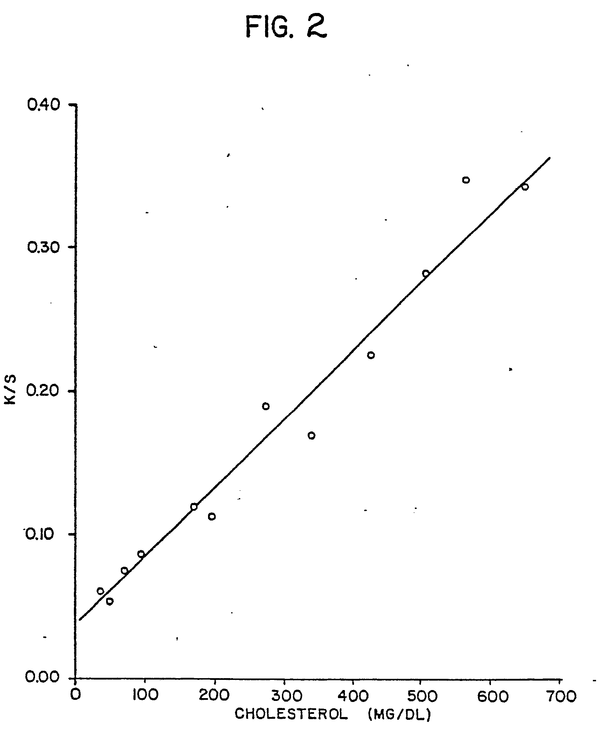

The concentration of cholesterol in each of the test solutions was proportional to K/S. The plot of K/S vs. cholesterol concentration is shown in Figure 2.

The resultant data show the integral analytical element to provide a quantitative detectable response to cholesterol concentration over a clinically significant range.

**Claims**

1. A device for determining an analyte in a liquid sample, which device (10) consists essentially of a single, integral matrix comprising: (a) a plurality of solid, reflective, inorganic particles (22); (b) each of said particles being coated with at least one substance (24) to provide a detectable response to said analyte; and (c) a binder (26) which is selectively permeable to said analyte or a reaction product thereof; characterised in that the device comprises two analyte responsive components, the second of which is said at least one substance coated on said particles and the first of which is separated therefrom by said binder, and wherein said first component is responsive to the analyte to form a reaction product, and the second component effects a detectable response to the reaction product.

2. A device according to claim 1, wherein the first component is reactive with the analyte to produce hydrogen peroxide and the second component includes a peroxidatively active substance and a redox indicator and is reactive with hydrogen peroxide to produce a detectable response.

3. A device according to claim 2, for determining cholesterol, wherein the first component includes cholesterol oxidase and cholesterol ester hydrolase.

4. A device according to claim 2, for determining uric acid, wherein the first component includes uricase.

5. A device according to claim 2, for determining glucose, wherein the first component includes glucose oxidase.

6. A device according to any of claims 1 to 5, wherein the particles have a diameter of from 0.1 to 200 μm, and are basic lead hydroxide, basic lead carbonate, titanium dioxide, zinc oxide, magnesium oxide, lead oxide, barium sulfate, calcium sulfate, strontium sulfate, silica, calcite, silicates and aluminosilicates, zinc sulfide, diatomaceous earth, clay or any mixture of two or more thereof.

7. A device according to any of claims 1 to 6, wherein the binder comprises at least one macromolecular substance which is a protein, gum or wax, or a polymer selected from homopolymers and copolymers of cellulose acetate, polyvinyl chloride, polyamide (nylon), starch, polycarbonate, polyvinyl alcohol, polyvinyl acetate, polyvinyl pyrrolidone, polyurethane, cellulose, cellulose nitrate, polysulfone and cellulose acetate butyrate.

**Patentansprüche**

1. Vorrichtung zur Bestimmung eines zu analysierenden Bestandteils in einer Flüssigkeitsprobe, wobei die Vorrichtung (10) im wesentlichen aus einer einzigen, ein Ganzes bildenden Matrix besteht, die (a) eine Anzahl fester, reflektierender, anorganischer Teilchen (22), von denen jedes (b) mit mindestens einer Substanz (24) überzogen ist, um ein erfaßbares Ansprechen auf den zu analysierenden Bestandteil zu erzielen, und (c) ein Bindemittel (26), das gegenüber dem zu analysierenden Bestandteil oder einem Reaktionsprodukt davon selektiv durchlässig ist, enthält, dadurch gekennzeichnet, daß die Vorrichtung zwei auf den zu analysierenden Bestandteil ansprechende Komponenten enthält, von denen die zweite diejenige Substanz ist bzw. diejenigen Substanzen sind, mit der bzw. denen die Teilchen überzogen sind, und von denen die erste durch das Bindemittel von der zweiten Komponente getrennt gehalten wird, wobei die erste Komponente auf den zu analysierenden Bestandteil unter Ausbildung eines Reaktionsproduktes anspricht und die zweite Komponente ein erfaßbares Ansprechen auf das Reaktionsprodukt bewirkt.

2. Vorrichtung gemäß Anspruch 1, dadurch gekennzeichnet, daß die erste Komponente mit dem zu analysierenden Bestandteil zu Wasserstoffperoxid umsetzbar ist und die zweite Komponente eine peroxidativ aktive Substanz und einen Redoxindikator umfaßt und mit Wasserstoffperoxid unter Erzeugung eines erfaßbaren Ansprechens umsetzbar ist.

3. Vorrichtung gemäß Anspruch 2 zur Bestimmung von Cholesterin, dadurch gekennzeichnet, daß die erste Komponente Cholesterinoxydase und Cholesterinesterhydrolase enthält.

4. Vorrichtung gemäß Anspruch 2 zur Bestimmung von Harnsäure, dadurch gekennzeichnet, daß die erste Komponente Uricase enthält.

5. Vorrichtung gemäß Anspruch 2 zur Bestimmung von Glucose, dadurch gekennzeichnet, daß die erste Komponente Glucoseoxidase enthält.

6. Vorrichtung gemäß einem der Ansprüche 1 bis 5, dadurch gekennzeichnet, daß die Teilchen einen Durchmesser von 0,1 bis 200 μm besitzen und aus basischem Bleihydroxid, basischem Bleicarbonat, Titandioxid, Zinkoxid, Magnesiumoxid, Bleioxid, Bariumsulfat, Calciumsulfat, Strontiumsulfat, Siliciumdioxidcalcit, Silicaten und Aluminosilikaten, Zinksulfid, Diatomeenerde, Ton oder einem Gemisch aus zwei oder mehreren dieser Stoffe besteht.

7. Vorrichtung gemäß einem der Ansprüche 1 bis 6, dadurch gekennzeichnet, daß das Bindemittel mindestens eine makromolekulare Substanz enthält, die ein Protein, Gummi oder Wachs oder ein Polymerisat ist, das aus Homo- und Mischpolymerisaten von Celluloseacetate, Polyvinylchlorid, Polyamid (Nylon), Stärke, Polycarbonat, Polyvinylalkohol, Polyvinylacetat, Polyvinylpyrrolidon, Polyurethan, Cellulose, Cellulosenitrat, Polysulfon und Cellulose-acetat-butyrat ausgewählt ist.

**Revendications**

1. Dispositif de dosage d'un analyte présent dans un échantillon liquide, lequel dispositif (10) est essentiellement composé d'une matrice intégrante unique comprenant: (a) une série de particules inorganiques solides, réfléchissantes (22); (b) chacune desdites particules étant revêtue d'au moins une substance (24) destiné à fournir une réponse décelable audit analyte; et (c) un liant (26) sélectivement perméable audit analyte ou à un produit de réaction de celui-ci; caractérisé en ce que le dispositif comprend deux constituants sensibles à l'analyte, dont le second est la ou chaque susdite substance revêtant lesdites particules et dont le premier est séparé de celui-ci par ledit liant, et dans lequel ledit premier constituant est sensible à l'analyte en sorte de former un produit de réaction, et le second constituant fait apparaître une réponse décelable au produit de réaction.

2. Dispositif selon la revendication 1, dans lequel le premier constituant est de nature à réagir avec l'analyte pour donner du peroxyde d'hydrogène et le second constituant comporte une substance à activité de peroxydase et un indicateur d'oxydo-réduction et est de nature à réagir avec le peroxyde l'hydrogène pour donner une réponse décelable.

3. Dispositif selon la revendication 2, pour le dosage de cholestérol, dans lequel le premier constituant comporte de la cholestérol oxydase et de la cholestérol ester hydrolase.

4. Dispositif selon la revendication 2, pour le dosage d'acide urique, dans lequel le premier constituant comporte de l'uricase.

5. Dispositif selon la revendication 2, pour le dosage de glucose, dans lequel le premier constituant comporte de la glucose oxydase.

6. Dispositif selon l'une quelconque des revendications 1 à 5, dans lequel les particules ont un diamètre de 0,1 à 200 µm, et sont de l'hydroxyde de plomb basique, du carbonate de plomb basique, du dioxyde de titane, de l'oxyde de zinc, de l'oxyde de magnésium, de l'oxyde de plomb, du sulfate de baryum, du sulfate de calcium, du sulfate de strontium, de la silice, de la calcite, des silicates et aluminosilicates, du sulfure de zinc, de la terre d'infusoires, de l'argile ou tout mélange de deux ou plusieurs de ceux-ci.

7. Dispositif selon l'une quelconque des revendications 1 à 6, dans lequel le liant comprend au moins une substance macromoléculaire qui est une protéine, une gomme ou une cire, ou un polymère choisi parmi les homopolymères et copolymères d'acétate de cellulose, de chlorure de polyvinyle, de polyamide (nylon), d'amidon, de polycarbonate, d'alcool polyvinylique, d'acétate de polyvinyle, de polyvinyl pyrrolidone, de polyuréthanne, de cellulose, de nitrate de cellulose, de polysulfone et d'acétobutyrate de cellulose.

FIG.1

# FIG. 2